# EUROPEAN PATENT SPECIFICATION

(11) **EP 0 862 421 B1**
(45) Date of publication and mention of the grant of the patent: **31.07.2002**
(21) Application number: 96939871.8
(22) Date of filing: 21.11.1996
(51) Int. Cl.: A61K 9/16

(54) **PROMPT-RELEASE PHARMACEUTICAL COMPOSITIONS**
PHARMAZEUTISCHE ZUBEREITUNG MIT SCHNELLER FREISETZUNG
COMPOSITIONS PHARMACEUTIQUES A LIBERATION RAPIDE

(30) Priority: 22.11.1995 IT MI952427
(43) Date of publication of application: 09.09.1998
(73) Proprietor: RECORDATI S.A. CHEMICAL and PHARMACEUTICAL COMPANY, 6830 Chiasso (CH)
(72) Inventor: SANTUS, Giancarlo, I-20146 Milano (IT); GOLZI, Roberto, I-26100 Cremona (IT)
(74) Representative: Gervasi, Gemma, Dr.
(86) International application number: EP9605127
(87) International publication number: WO9718798

(56) References cited:
- EP-A- 0 068 450
- EP-A- 0 273 890
- US-A- 4 764 375
- US-A- 5 296 236

## Description

### FIELD OF INVENTION

The present invention concerns a pharmaceutical composition which promptly releases the active principle, suitable in particular for oral administration. The present composition comprises coated nuclei containing one or more active ingredients, which may be for istance used in the preparation of extemporaneous suspensions, as well as of tablets or solid aggregates.

Suspensions are heterogeneous systems in which the continuous phase (external phase) is liquid or semisolid, while the dispersed phase (internal phase) consists of solid particles that are insoluble in the medium used. Pharmaceutical suspensions can be prepared during the industrial production phase (ready-to-use suspensions), or prepared by the patient at the time of use (extemporaneous suspensions).

The suspension is one of the most versatile of the various pharmaceutical forms that can be used for administration, since it is suitable for the creation of preparations for oral, injectable, and dermatologic use.

The use of suspensions for the administration of active ingredients has been known for some time, and ready-to-use oral suspensions are usually preferred in everyday practice. This preference is due to the fact that the patient must simply agitate the bottle prior to use, since the continuous liquid phase is already present in the bottle. To take an extemporaneous suspension, however, the patient must first redisperse the powdered drug in water.

Examples of ready-to-use suspensions already on the market are Bactrim (R), a syrup with antibacterial action, and Maalox (R), a suspension with antacid action used to treat epigastric pain.

The preparation of these administration forms in quantity, with consistent and repeatable standard characteristics, is normally influenced by a large number of variables, for example the density of the internal and external phases; the ratio of the phase volumes; the viscosity of the external phase; and the dimensions, degree of aggregation, and shape of the particles. The variability of these parameters therefore causes difficulties in resuspension, even after agitation at the time of use, and in some cases can lead to inhomogeneity in the distribution of the active ingredient.

In addition, currently available suspensions can have certain limitations which make them poorly suitable for use. This occurs, for example, when problems exist with instability of the vehicle or palatability of the suspended form, when active ingredients with unpleasant organoleptic characteristics are used, or when mutually incompatible active ingredients are present simultaneously in the same formulation.

To eliminate these drawbacks, measures have been known for some time which tend to mask the active ingredient, isolating it from the ingestion medium.

One of the measures most frequently used is that of microencapsulation, which however implies the use of solvents and of a process that is costly in industrial terms. Another drawback of masking performed by means of microencapsulation consists in the delaying action that the encapsulating substances can exert with regard to release of the active ingredient.

On the other hand, in some cases this delaying effect has been used specifically in order to obtain a modulation of the release of the drug according to predetermined profiles. For example, US Patent 5,296,236 describes microgranular controlled-release suspensions for oral use which have the peculiarity of gradually releasing various types of active ingredient over time, thus adapting them to the various desired therapeutic conditions.

A delay in the release of the active ingredient may, however, not always be desirable or necessary for the administration of certain active ingredients such as, for example, analgesics, antipyretics, antitussives, and the like.

Another advantage of suspensions consists in their particular ease of swallowing (typical of liquids), especially when compared to solid pharmaceutical forms.

It is in fact known that, especially in pediatrics and in the elderly, and in particular categories of patients suffering from motor coordination impairment, such as stroke victims, it can be difficult to take tablets.

The need to respond to this kind of problem is illustrated by the continuing search for devices making it easy to break a tablet to produce a powder that can be dispersed in water.

For example, international patent applications WO 95/6427 and WO 95/6428 have recently described devices in the form of a syringe or superimposed cups, capable of allowing breakage of the tablet and subsequent resuspension thereof in water. Such devices have obvious limitations, however, for example in the presence of poorly palatable active ingredients, leaving aside the fact that very often the excipients of the tablets are not among those most suitable for promoting suspension of the crushed tablet.

A particular need is therefore felt not only for drugs that are easily available in suspended form, but also, when necessary, for the ability to mask taste.

A number of active ingredients have such palatability problems. It is well known, for example, that the bitter and metallic taste of acetaminophen, a very common analgesic and antipyretic, makes it difficult to administer this drug by means of liquid formulations, especially in pediatrics. Other examples consist of naproxen, a well-known anti-inflammatory characterized by the fact that it leaves an intense burning sensation in the mouth and of ibuprofen; diltiazem, a cardiovascular drug with a strongly bitter taste; and moguisteine, a bitter-tasting antitussive with anesthetic properties.

In the case of diltiazem in particular, the product is also highly unstable in aqueous solution, and it is therefore very advantageous to have available an extemporaneous suspension that has good stability as well as a pleasant taste.

The need has also been felt for formulations which control the hygroscopicity of certain substances. For example, it is known from the literature that potassium bicarbonate can be used to prevent osteoporosis and hypertension [New Engl. J. Med. 330, 1251, 1776 (1994), and US Patent 5,171,583]. However, since this salt has tolerability and hygroscopicity problems, the ability to have available a pharmaceutical form which can overcome these drawbacks without at the same time controlling release of the active ingredient constitutes a good solution to the problem.

An object of the present invention is therefore to produce formulations in extemporaneous suspension, in particular for oral use, which, while allowing prompt release of the active ingredient, at the same time avoids those aforementioned problems that can be encountered with common ready-to-use suspensions.

In addition, the present coated nuclei, preferably the coated microgranules, can be formed into tablets or solid aggregates obtained for instance by lyophilizing a mixture of coated nuclei and of a vehicle of excipients, that can be administered without water. Thus there is no need of reconstitution by admixing them with a liquid phase before administration. The patient itself provides the fluid, e.g., saliva.

### SUMMARY

The present invention is represented by a Prompt-release pharmaceutical composition for administration of an active ingredient which is released in an amount of no more than 10% by weight within 1 minute from mixing with the aqueous reconstitution phase and of at least 75% by its weight within 45 minutes from mixing with the aqueous reconstitution phase, said composition comprising:
a) a plurality of nuclei represented by microgranules having dimensions between 50 and 500 micrometers, containing at least one active ingredient and at least one pharmaceutically acceptable excipient;
b) a film coating applied onto said microgranules, consisting of a lipidic layer, sprayed in the melted state onto said microgranules, with no aid of solvents, said coating optionally comprising at least one hydrophilic additive, either incorporated into the lipidic layer or as separate hydrophilic layer applied onto said lipidic layer, provided that said coating contains 1% - 25% by weight of lipid material with respect to the weight of microgranules to be coated;
c) a vehicle comprising one or more pharmaceutically acceptable excipients.

Other aspects of the present invention are represented by the the coated nuclei, and by the the process of preparation of the present compositions and/or coated nuclei. Other objects of the present inventions will be apparent from the present description text.

It has been found in this context that by applying lipid materials in the melted state by film-coating, optionally in the presence of hydrophilic additives which act as wetting elements, it is possible to obtain formulations suitable for extemporaneous suspension and capable of masking the taste of the active ingredient and improving its stability characteristics.

In particular, the present invention comprises coating microgranules or crystals of active ingredient, having dimensions between 50 and 500 µm and preferably between 100 and 300 µm, with lipid material in the melted state, such that the coated micronuclei can form a suspension which can be reconstituted by the patient immediately before use by simply adding the suspending phase, or formed into tables or solid aggregates.

The suspension, reconstituted at the time of use by admixing the coated nuclei and the vehicle with optionally the liquid suspending phase, is designed to allow release of the active ingredient only a few minutes after ingestion. It is thus possible to have in suspension mixtures of different active ingredients, even those chemically incompatible with one another, and to have preparations with good flavor masking, but without release of the active ingredient being substantially influenced by the formulation. Specifically, such release occurs within a time from reconstitution not exceeding 60', depending on the greater or lesser solubility of the active ingredient in the suspending phase.

By means of the invention, the assimilation conditions of the suspension thus mimic the ingestion conditions of a film-coated tablet which normally requires a time varying from 30 to 60' before the active ingredient can be solubilized and absorbed. The average percentage dissolution of a tablet is estimated by many pharmacopeias to be equal to 75% within a period of 45' [U.S. Pharmacopeia XXIII, p. 1925].

It is obvious that a liquid containing the active ingredient in the form of granules of reduced dimensions, coated with lipid material, is easier to swallow as compared to a tablet.

### PRIOR ART

The use of waxes to coat tablets, to mix with powders, or for preparing drug/melted wax granulates, has been known for some time in the prior art.

Waxy materials have been used to form waxy matrices of tablets or granulated materials, principally for the purpose of obtaining controlled-release formulations [Pharm. Acta Helv., 56 4/5, 111 (1981)].

US Patent 4,764,375 AND EP 273,890 describe the possibility of obtaining crystals of soluble active ingredients that are directly incorporated into melted waxes, and the possibility of forming extemporaneous suspensions with or without the addition of surfactants.

Patent application EP 608,850 discloses how to form microgranulated materials having characteristics such that they can easily be suspended in aqueous solutions after film-coating.

### DETAILED DESCRIPTION OF THE INVENTION

The present invention typically applies to active ingredients endowed with palatability and/or taste problems, with poor stability in the admnistration vehicle, or with hygroscopicity problems, such as the drugs hereinabove mentioned in the present application.

Other examples of active ingredients which may be included in the coated nuclei suitable for use according to the present invention are diphenylhydramine hydrochloride or citrate, prednisone, fluoxetine, fluconazole, paroxetine, ketoprofen, dextromethorphane hydrobromide.

Typically, the nuclei to be coated according to the present invention are microgranules, and they are preferably obtained by wet granulating one or more active ingredients with one or more pharmaceutically acceptable excipients.

Suitable excipients are for instance binders, fillers, lubricants, aromatizing agents, buffering agents, antioxidants agents and mixtures thereof.

For instance, the binder can be polyvinylpyrrolidone, polyvinylpyrrolidone/vinyl acetate copolymer, cellulose derivatives (esters, ethers), and mixture thereof; the fillers can be dibasic calcium phosphate, lactose, microcrystalline cellulose, starch, sugars, or mixtures thereof; the lubricants can be talc or magnesium stearate; the buffering agents can be citric acid and tartaric acid.

The fluids typically used for granulating the active ingredients with the excipients, hereinafter referred to as binding or granulating fluids, can be water or organic solvents, such as ethyl alcohol or other commonly used solvents, or any mixture thereof.

According to a typical embodiment of the present invention, the nuclei to be coated are microgranules obtained by wet granulating the active ingredient with a mixture of a filler, such as lactose, and of a binder, such as polyvinylpirrolidone, and optionally also of a plasticizing agent, such as for instance polyethyleneglycole. The granulating fluid is typically water.

Preferably, the microgranules to be coated according to the present invention are wet granulated in a high-shear mixer-granulator equipped with a mixer and a mill, wherein the mixture of the active ingredients and of the excipients is wetted with a binding fluid then kneaded, wetting and kneading being both carried out under the combined action of the mixer and of the mill. The amount of binding fluid can be for instannce of about 20-50 grams per kilogram of dry mixture of active ingredients and of excipients.

In the present text, dryness levels typically correspond to a residual water content lower than about 10% by weight, preferably lower than about 5%-8% by weight.

The various active ingredients that can be used according to the present invention are preferably converted into microgranular nuclei specifically according to the procedure described by application EP 608,850, which is herein incorporated by reference. For the purposes of the invention, it is extremely important to have available microgranutated materials with morphological characteristics suitable both for subsequent coating and for producing a good suspension.

To prevent the lipid material applied onto the nuclei from exerting an excessive delaying effect on release of the active ingredient, it is therefore spray-deposited onto the microgranulated material itself in layers of greater or lesser thinness depending on the differing solubility of the active ingredient.

Preferably, the lipidic coating according to the present invention is in amounts such as to not substantially affect the final dimensions of the particles to be coated, which preferably remain within the size ranges hereinafter reported for the nuclei to be coated.

In theory, if the active ingredient is present in homogeneous form, with crystals of regular shape with no sharp angles, the lipid material could be applied directly onto the crystals of active ingredient. It is obvious, however, that this kind of situation, although not impossible, is quite specific and may be encountered only in a very limited number of cases.

This problem can be overcome, however, in the case of the present invention, since it will always be possible to resort to microgranulation of the active ingredient in order to make the coating process applicable to any active ingredient regardless of its crystalline and morphological characteristics.

In principle, various techniques can be used for coating nuclei. Some of these, however, exhibit limitations that make industrial use impractical.

The possibility is thus known, for example, of performing coatings in rotating tanks. This technology cannot, however, be applied successfully to the coating of microgranulated materials having dimensions between 100 and 300 um, due to problems of agglomeration between the particles being coated.

The microencapsulation technology, on the other hand, which has already been successfully applied for direct coating of active ingredients (U.S. Patent 4,822,619), requires the use of solvents and is difficult and complex to standardize.

It has been found that in the case of the present invention, the most suitable technology consists in coating the nuclei, preferably the aforementioned microgranules in fluidized-bed equipment by means of the Wurster^{R} system [Pharm. Res 7 (11), 1119(1990)].

In this system, the lidipic coating material is sprayed in melted state through a nozzle onto the particles which are held suspended by a stream of air inside a cylinder. After exposure to the coating material, the particles have the opportunity to decelerate in an expansion chamber, and to drop to the bottom of the device to begin a new film-coating cycle.

Among the various materials appropriate for coating, lipid materials are those found to be most suitable for achieving the goal of obtaining coated particles such that release of the active ingredient begins predominantly after 1 minute (preferably, no more than 10% by weight of the active ingredient is released within 1 minute from reconstitution of the liquid suspension or from ingestion of the tablets or solid aggregates), and is predominantly complete within 45 minutes (preferably, at least 75% by weight of the active ingredient is released within 45 minutes from reconstitution of the liquid suspension or from ingestion of the tablets or solid aggregates).

It is moreover known that lipid materials, in addition to being hydrophobic, are poorly soluble in most common organic solvents usable in the pharmaceutical field. Optimum solvents would thus be the chlorinated ones, such as chloroform, dichloroethane, carbon tetrachloride, and the like, although they have considerable limitations as far as toxicological and environmental aspects are concerned.

It has therefore been found, and is a part of the present invention, that by adapting so-called melted-wax technology to said materials, it is possible to apply a layer of lipid material onto the nuclei in a controlled manner the lipidic material being atomized in the melted state, using only preheated compressed air as the atomization fluid. In this manner the lipid materials, which are kept molten, are atomized in the desired quantity, without the aid of any solvent, onto the nuclei containing the active ingredient.

According to a typical embodiment of the present invention, the lipidic material is preferably melted at a temperature of about 40°C-60°C above its melting point, then sprayed onto the nuclei at a temperature preferably of about 20°C-30°C above its melting point, at a spraying rate of preferably 2.0-4.0 grams/minute, more preferably of 2.5-3.5 grams/minute.

Compressed air used as the atomization fluid is preheated, typically at a temperature of about 40°- 60°C above the melting point of the selected lipidic material, and atomized at a pressure preferably of about 2.5-3.5 bars, typically of about 3 bars.

Coated microgranules are then cooled, preferably to about 10-15°C below the melting point of the lipidic material, for not less than 10 minutes, and then further cooled till they reach the room temperature (25-30°C).

Lipid materials suitable for the purpose, usable alone or mixed, are:
- Mono-, di-, and triglycerides of fatty acids having from 6 to 32, preferably from 12 to 22, typically 16 or 18 carbon atoms, principally saturated, such as: Monostearin, dipalmitin, tristearin, hydrogenated castor oil (Sterotex) (R);
- Fatty acids or fatty alcohols having from 6 to 32, preferably from 12 to 22, typically of 16 or 18 carbon atoms, such as: stearic acid, cetyl alcohol, stearyl alcohol;
- Esters of propylene glycol or of sucrose with fatty acids having from 6 to 32, preferably from 12 to 22, typically of 16 or 18 carbon atoms, such as: propylene glycol monostearate, sucrose monostearate, and sucrose monopalmitate;
- Waxes, such as: beeswax white wax (a refining product obtained by treating with oxidizing agents natural, yellow beeswax), candelilla wax, carnauba wax, and the like.

Typically, the amount of lipidic material applied to the nuclei is of about 1%-25% by weight, and preferably of about 5%-20% by weight, with respect to the weight of dry nuclei (microcrystals or microgranules) to be coated (dryness levels being preferably as hereinabove reported in the present text for microgranules).

It has also been found, and this also forms part of the present invention, that the wettability of the present coated microgranules is further improved by adding a hydrophilic additive to the lipidic coating, thus further reducing tendency of suspended particles, if any, to float and/or to adhere to the walls of the reconstitution container.

Suitable hydrophilic materials are the hydrophilic polymers, in particular: water soluble polymers, for instance semisynthetic ones, such as cellulose derivatives (ethers, esters) such as: cellulose acetophthalate, hydroxypropylmethylcellulose [Methocel®], hydroxyethylcellulose, hydroxypropylcellulose, or synthetic polymers such as, polyethylene glycol, and the like. It is important in any case to apply the polymer in quantities such as not to create gastroresistance or release control beyond the desired limits. This quantity of hydrophilic element may lie in the range between 0.1% and 5%, preferably between 0.5 and 2%, by weight of hydrophilic component with respect to the lipid coating material.

The hydrophilic additive can be either incorporated into the lipidic layer deposited onto the nuclei (e.g. by dispersing the hydrophilic additive into the melted lipidic material, prior to spraying it onto the nuclei), or applied as separate layer onto the lipidic layer (e.g. by spraying a solution thereof in appropriate solvent onto the nuclei already coated with a lipidic layer).

Other examples of hydrophilic additives suitable for the purpose are ionic surfactants for instance anionic surfactants, among them those containing carboxylate, sulfonate, or sulfate groups such as dioctyl sodium sulfosuccinate or sodium lauryl sulfate, or nonionic surfactants such as partial esters of fatty acids with anhydrides of sorbitol (Span) or with polyoxyethylene ethers of fatty acid partial esters of sorbitol anhydrides (Tween).

Fatty acids have from 6 to 32, preferably from 12 to 22, and typically 16 or 18 carbon atoms.

The lipidic coating according to the present invention achieves the goals of providing sufficient masking effect of unpleasant taste and/or protective effect towards degradation of active ingredient, and a satisfactory release pattern of the active ingredient, using amounts of lipidic material sufficiently low to allow the dimensions of the coated nuclei to be preferably maintained within the prescribed limits hereinabove reported, and to be applied onto the nuclei in a uniform manner, so to produce coated nuclei as smooth as possible, suitable for suspensions in liquid medium.

According to a preferred embodiment of the present invention, the lipidic material to be applied to the nuclei comprises at least one mono-glyceride or di-glyceride of a fatty acid (preferably a mono-glyceride, such as glyceryl monostearate) and at least one wax (such as beeswax, white wax or candellilla wax); more preferably, such lipidic material also comprises at least one fatty alcohol, such as cetyl alcohol, stearyl alcohol, or mixtures thereof, typically a mixture of cetylic and stearyl alcohol.

In particular, a typical lipidic coating according to the present invention contains, with respect to the total weight of lipidic material: about 40%-95% by weight, typically about 80%-90% by weight, of a mono-glyceride or di-glyceride of fatty acids, preferably of a mono-glyceride of fatty acids; about 5%-50% by weight, typically about 5%-20% by weight, and more preferably about 5%-10% by weight, of a wax; preferably, such lipid mixture may optionally further comprise about 0.5%-5% by weight, typically 1%-3% by weight of one or more fatty alcohols; typically the amount of such lipidic coating is of about 10% by weight with respect to the nuclei to be coated.

For instance, a preferred lipidic coating according to the present invention may contain about 90% by weight of glyceryl monostearate, about 8% of a wax, about 1% by weight of cetyl alcohol, and about 1% by weight of stearyl alcohol.

According to a further preferred embodiment of the present invention, the lipidic material used to coat the nuclei further comprises 1%-3% by weight of a hydrophilic polymer, such as hydroxypropylmethyl cellulose, or 2%-3% by weight of a surfactant, such as dioctyl sodium solfosuccinate or sodium lauryl sulfate (percentages being referred to the total amount of lipidic coating).

According to another embodiment of the present invention, the lipidic coating comprises at least one trygliceride, such as hydrogenated castor oil, and preferably it also contains about 0.5%-2% by weight of an hydrophilic additive, such as polyethylene glycole, percentages being referred to the total amount of lipidic material.

Typically, the lipidic mixture is melted at about 110°C-130°C, then sprayed onto the nuclei to be coated at about 80°C, at a spraying rate preferably of about 2.5-3.2 grams/minute, using compressed air preheated preferably at about 110°C-130°C, with a pressure preferably of about 3 bars.

Once coated in this manner, the microgranulated material containing the active ingredient can be further formulated with the addition of one or more other excipients which represent the "vehicle," which will then be reconstituted with the external phase prior to use in a suitable container.

The vehicle can be either admixed with the coated nuclei, and stored in dosage forms, or added to the liquid suspending phase. In the former case, such vehicle of excipients is typically in solid form, and it does not allow any substantial release of the active ingredient in it. If desired, the mixture of the coated nuclei with the vehicle can be lyophilized, so to ensure the active ingredient to be stored in a sufficiently dry form.

The constituents of the vehicle can be selected among the following ones and any mixture thereof:
- Suspending or structuring agents such as: cellulose esters, microcrystalline cellulose, alginic acid derivatives, polyvinylpyrrolidone or derivatives;
- Sugars, which represent the substrate of the said vehicle, such as: sucrose, sorbitol, xylitol, dextrose, mannitol, and the like;
- Buffering substances such as: citric acid, sodium citrate, sodium phosphate and potassium phosphate, and the like;
- Flavors and edulcorants such as: saccharine, aspartame, and flavors commonly used in the pharmaceutical sector.

Substances to reinforce the taste, such as citric acid, sodium chloride, and the like, can also be present.

Types and amounts of excipients constituting the vehicle, either in the case of suspensions, or of tablets or solid aggregates, may vary as a function of the type and solubility of the active ingredient, the vehicle being typically in weight amounts of from 5 to 10 times the weight of the coated nuclei.

Typically, the vehicle comprises a sugar (e.g. sucrose, optionally in admixture with mannitol), generally admixed with a suspending or structurating agent (such as polyvinylpirrolidone), and optionally with other excipients, such as flavoring agent (such as lemon flavour), a buffering substance (such as citric acid), and other excipients such as precipitated silica.

According to a typical embodiment, such vehicle contains about 80%-98% by weight of one or more sugars (typically a mixture of sucrose and of mannitol, in weight rations of about 8:1 to 5:1) and about 1%-2% by weight of a structurating agent (for istance polyvinylpirrolidone); optionally, such vehicle may also contain about 1%-2% by weight of a flavoring agent, about 2%-4% by weight of a buffering substance, and about 0.1%-0.2% of silica gel.

Before being reconstituted with the appropriate external phase by the patient, the mixture intended for suspension, represented by the coated nuclei, optionally admixed with said vehicle can be distributed into various dosage forms.

Particularly convenient is the single-dose packet, generally consisting of a paper/aluminum/polyethylene laminate. Laminates having 10 g/m² paper, aluminum at a thickness of 10 to 20 um, and 30 g/m² polyethylene can be used for packets.

Another single-dose dosage form consists of reservoir-stopper vials. They have the advantage that the vehicle is already present in them in the liquid phase contained in the bottle, while the coated nuclei (preferably said microgranules) are stored in the reservoir stopper. The user, having removed a protective ring, can breach the reservoir stopper by pressing on it, causing the microgranules to drop into the part below containing the liquid vehicle.

For administrations which require multiple doses, a dosage form can be used comprising a multi-dose bottle containing a mixture of coated nuclei (preferably said microgranules) and the vehicle. The user can measure out this mixture using appropriate solid measures, thus reconstituting the necessary quantity of suspension.

A mixture of several active ingredients can be present simultaneously in the same dosage form.

The pharmaceutical composition according to the present invention allows the quantity of the various components of the mixture of active ingredients present in the same dosage unit to be modulated as a function of a specific, personalized therapeutic treatment.

The external phase used as suspending medium is typically an aqueous phase, and may be either added by the user (e.g. potable water used by the patient to dissolve the drug), or included in the pharmaceutical composition, e.g. a suspending phase which may contain one or more excipients, including those hereinabove mentioned for the vehicle.

In addition, the coated nuclei, preferably the coated microgranules, can be formed into dosage unit forms such as tablets or solid aggregates, typically lyophilized, and the like. These can be administered without water or the need for reconstitution. The patient itself provides the fluids (saliva or gastrointestinal fluids). The ingredients usually employed in the preparation of the tablets include excipient materials like those described above and also include: mannitol, dextrins, sugars, etc. as diluents in presence of binders such as, for example, gelatin or polyvinylpyrrolidone (PVP), and the like. Flavoring agents and edulcorants can also be added to these materials.

It has also been found that the inclusion of an effervescent mixture in the lyophilized tablet or solid aggregates is particularly useful in further reducing the sand effect of the microparticles Materials suitable for preparing an effervescent mixture are bicarbonates and organic acids such as citric acid or tartaric acid and the like. The effervescent tablets or solid aggregates are then lyophilized in presence of organic solvents, such as: dioxane, terbutyl alcohol, etc.

An assessment of the release time of the active ingredient of the formulation was made by dispersing the microgranulated material, coated with lipid material, in water, then taking a sample of the solution at different times and determining the amount of active ingredient contained in it. The microgranulated material had to be coated with lipid material in such a way that during the time needed to reconstitute it with water (approximately a minute) there was no release of active ingredient, and that release was complete after 45 minutes, in order to avoid slow-release phenomena which are not within the context of the present invention.

Several examples are supplied below, with the sole purpose of better illustrating the invention in question by demonstrating its advantages and applicability, but without constituting any limitation thereof.

### EXAMPLE 1

### Microgranular formulation of potassium bicarbonate

2 kg of potassium bicarbonate was mixed with lactose and polyvinylpyrrolidone K 30, and wetted with 100 ml of water in a Fielder P25 granulator. After adding the water and granulating for 10', the granulated material was dried and sieved until a granule fraction was obtained having dimensions between 100 µm and 300 µm. A mixture of lipids having the following composition was then applied onto the granulated material:

| **Component** | **% (w/w)** |
|---|---|
| Beeswax | 8 |
| Glyceryl monostearate | 90 |
| Cetyl alcohol | 1 |
| Stearyl alcohol | 1 |

The lipids were first melted at a temperature of approximately 110°C, and then sprayed in the melted state, with the temperature maintained at approximately 80°C, using compressed air preheated to a temperature of 125°C and at a pressure of 3 bar. The spraying process is performed with a 7" Wurster insert on a Glatt GPCG 3 apparatus. The quantity of lipid material was equal to 15% w/w with respect to the granulated material, and it was sprayed at a rate of 2.5 g/min. In the final phase of the coating process, sodium lauryl sulfate in a quantity equal to 1% of the weight of the lipids used was dispersed in the melted lipids. The microgranulated material coated in this manner exhibited good dispersibility in water.

### EXAMPLE 2

### Tasteless, prompt-release microgranular compositions of diltiazem

Using the method described in Example 1, a microgranulated material having the following composition was prepared:

| **Component** | **Weight (g)** |
|---|---|
| Micronized diltiazem hydrochloride | 600 |
| Micronized lactose (Microtose (R)) | 2100 |
| Polyvinylpyrrolidone K30 | 300 |

After drying, the fraction of granules having dimensions between 125 and 300 um was separated by sieving. A Glatt GPCG 3 apparatus with a 7" Wurster insert and 1.2 mm nozzle was used to apply a composition consisting of the same mixture of components in two different formulations (I and II):

| **Component** | **Formula I Formula II (%)** | |
|---|---|---|
| Glyceryl monostearate | 90 | 49 |
| White wax | 8 | 49 |
| Cetyl alcohol | 1 | 1 |
| Stearyl alcohol | 1 | 1 |

The lipid components were melted at a temperature greater than 80°C, and were then sprayed with a pump having a flow rate of 3.2 g/minute and an atomization pressure of 3 bar, using compressed air preheated to a temperature of 120°C.

The following nine different coating tests (A-I) were performed; indicated for each is the percentage of coating material and the respective percentage of surfactant (dioctyl sodium sulfosuccinate - DSS) used, where applicable.

| | A | B | C | D | |
|---|---|---|---|---|---|
| Formula I | 10% | 15% | 20% | 20% | |
| DSS | - | - | - | 0.2% | |

| | E | F | G | H | I |
|---|---|---|---|---|---|
| Formula II | 5% | 10% | 15% | 20% | 20% |
| DSS | - | - | - | - | 0.2% |

In formulations D and I, the percentage of surfactant was calculated with respect to the waxes and was added at the end, incorporating it directly into the lipid material.

### EXAMPLE 3

### Dissolution tests on formulations containing diltiazem

The dissolution of formulations (A-I) of Example 2 was determined in accordance with the procedure cited in USP XXIII, p. 1787, using 900 ml of distilled water, and spectrophotometric reading at a wavelength of 240 nm. 1 ml of solution was taken respectively at 1', 30', and 45' from the beginning of the test. Table I below lists percentage values of diltiazem detected in solution for each of the formulations analyzed.

**TABLE I**

| % diltiazem released | | | | | | | | | |
|---|---|---|---|---|---|---|---|---|---|
| Time | A | B | C | D | E | F | G | H | I |
| 1' | 3.2 | 0.2 | 0 | 2 | 26 | 2.7 | 2 | 0.3 | 2 |
| 30' | 100 | 91 | 93 | 98 | 100 | 75 | 36 | 15 | 83 |
| 45' | 100 | 100 | 95 | 99 | 100 | 98 | 78 | 52 | 100 |

### EXAMPLE 4

### Preparation of single-dose packets of prompt-release diltiazem

3.45 kg of granular phase, coated according to formulation B of Examples 2 and 3, was mixed with a vehicle consisting of:

| **Component** | **Weight (kg)** |
|---|---|
| Mannitol | 5 |
| Polyvinylpyrrolidone | 0.5 |
| Lemon flavor | 0.5 |
| Citric acid | 0.9 |
| Precipitated silica | 0.05 |
| Sucrose to make | 30 |

The resulting mixture was measured into 10,000 3-gram packets each containing 60 mg of diltiazem hydrochloride, using a Marchesini packing machine. The composition of each packet was 30 g/m² paper, 12 um aluminum, and 40 g/m² polyethylene.

### EXAMPLE 5

### Microgranulating acetaminophen and coating to mask taste

The method described in Example 1 was used to prepare a microgranulated material having the following composition: 50% acetaminophen, 40% lactose, 10% polyvinylpyrrolidone (PVP K30). After granulation with water, drying, and sieving over a 0.6 mm mesh to separate the fraction between 100 and 300 µm, the resulting granulated material was coated under the conditions stipulated by Example 2 with a lipid mixture containing 80% glyceryl monostearate and 20% carnauba wax. The coated granulated material was mixed with a vehicle in such a way that after distribution into packets, each packet had the following composition:

| **Component** | **Weight (g)** |
|---|---|
| Granulated acetaminophen | 0.550 (equal to 0.250 g of acetaminophen) |
| Polysorbate 80 | 0.01 |
| E 110 coloring | 0.0016 |
| Citric acid | 0.1 |
| Orange flavor | 0.10 |
| Orange powder | 0.60 |
| Caraway flavor | 0.003 |
| Precipitated silica | 0.01 |
| Powdered sugar to make | 3 |

The polysorbate 80 was absorbed onto the precipitated silica before being mixed with the other excipients of the packet.

### EXAMPLE 6

### Prompt-release suspension of naproxen with taste masking

The method described in Example 1 was used to prepare a microgranulated material having the following composition: 3200 g micronized naproxen, 400 g polyvinylpyrrolidone (PVP K30), 400 g lactose, and 30 g polyethylene glycol (PEG 6000). After mixing for 5 minutes, 500 ml of water was added by spraying at 2 bar with agitation, and the mixture was mixed for another 15 minutes until the microgranules formed and spheroidized. Drying was performed for 2 hours at 35°C in a static oven to a residual moisture of between 4 and 5% by weight, and a 0.6 mm sieve was used to separate the fraction of microgranules between 90 and 300 µm. A lipid mixture having the following composition (Formula III) was applied to the resulting granulated material :

| | |
|---|---|
| Glyceryl monostearate | 90% |
| Beeswax | 8% |
| Cetyl alcohol | 1% |
| Stearyl alcohol | 1% |

The following six different coating tests (L-P) were performed, for which an indication is given of the percentage of coating material and the respective percentage of surfactant (Methocel (R) E15LV) added where applicable. In the case of formulations N, O, and P, the Methocel (R) was sprayed after having been dissolved in an 80:20 mixture of methanol and water.

| | L | M | N | O | P | Q |
|---|---|---|---|---|---|---|
| Formula III | 5% | 10% | 10% | 10% | 10% | 15% |
| Methocel (R) E15LV | - | - | 1% | 2% | 3% | - |

### EXAMPLE 7

### Dissolution tests for the formulations containing naproxen

Dissolution of the formulations L-Q was performed according to the method indicated in Example 3, using a pH 7.4 phosphate buffer as the medium and reading the spectrophotometric absorption at 330 nm. The results are indicated in Table II below.

**TABLE II**

| % naproxen released | | | | | | | |
|---|---|---|---|---|---|---|---|
| | Time | L | M | N | O | P | Q |
| | 1' | 0.3 | 0.2 | 0.2 | 0.5 | 0.5 | 0.5 |
| | 30' | 56 | 29 | 40 | 48 | 50 | 22 |
| | 45' | 75 | 55 | 63 | 80 | 85 | 40 |

### EXAMPLE 8

### Prompt-release suspension of ibuprofen with taste masking, for pediatric use

A mixture consisting of 400 g of micronized ibuprofen, 500 g of lactose, and 100 g of polyvinylpyrrolidone was mixed for 5 minutes and granulated with 210 ml water for 20 minutes. The mixture was then dried at 40°C in a fluidized bed for 10 minutes to a residual relative moisture of approximately 5% by weight. The fraction between 100 and 300 µm was separated, and a lipid composition (7,5% by weight with respect to the granulated material) was applied consisting of hydrogenates castor oil (Sterotex®) to which 1% polyethylene glycol (PEG 6000) had been added in the final film-coating phase. The microgranulated material coated in this manner was measured into the reservoir stopper of single-dose vials having as vehicle an 8-ml solution having the following composition:

| | |
|---|---|
| Sorbitol | 3500 mg |
| Avicel | 50 mg |
| Orange flavor | 50 mg |
| Citric acid | 15 mg |
| Sodium benzoate | 10 mg |
| Water to make | 8 ml |

Before use, the microgranular contents of the reservoir stopper (equal to 100 mg ibuprofen) were brought into contact with the liquid in the vial, and after agitation could be taken by the patient.

### EXAMPLE 9

### Extemporaneous suspension of moguisteine with taste masking

A mixture consisting of 49% moguisteine, 10% polyvinylpyrrolidone (PVP K30), 40% lactose, was mixed in a Fielder P25 kneader/granulator. A 5% aqueous solution of polyethylene glygol (PEG 6000), in amount corresponding to a PEG content of 1% by weight with respect to the total mixture as above defined, was added to the mixture at a rate of 25 ml/min, using a 0.8 mm nozzle and a spray pressure of 2 bar. Kneading proceeded for approximately 20' at a speed of 200 rpm, then the granulated material was allowed to spheroidize for another 15'. After drying, the granulated material was separated by sieving, yielding a fraction with dimensions between 100 and 300 µm. The microgranulated material was then coated with a lipid mixture having the following composition:

| | |
|---|---|
| Glyceryl monostearate | 90% |
| Beeswax | 8% |
| Cetyl alcohol | 1% |
| Stearyl alcohol | 1% |

1% by weight (with respect to the mixture) dioctyl sodium sulfosuccinate was added to this mixture in the final spraying phase by dispersion into the melted mass. The weight of the coating mixture was 5% of the weight of the microgranulated material being coated. The coated microgranulated material was then mixed with a vehicle such that after distribution into packets, each packet had the following composition:

| **Component** | **Weight (mg)** |
|---|---|
| Granular moguisteine | 430 (equal to 200 mg moguisteine) |
| Avicel (R) RC 591 | 500 |
| Ammonium glycyrrhizinate | 50 |
| Fruit flavor | 50 |
| Sucrose to make | 3000 |

### EXAMPLE 10

### Lyophilized tablet

Tablet having the following composition: coated granules placebo 50 mg, a vehicle containing the following excipients: gelatin 40 mg, mannitol 30 mg, aspartame 0.5 mg, citric acid 3.5 mg and also water 480 mg are prepared as follows:
the gelatin is dissolved in water heated at 60°C. Mannitol, citric acid and aspartame are added until solubilization is complete. The coated granules are added until an homogeneous suspension is obtained. The mixture is placed in blister pockets with 12 wells with a 6 mm diameter at the weight of 600 mg. Lyophilization is performed by freezing at about -40°C to about -45°C for 2-3 hours, then gradually raising the temperature up to about 25°C under a vacuum of about 0.2-0.25 mbar. After lyophilization is completed, the blister is sealed with an aluminium foil.

### EXAMPLE 11

### Effervescent tablet

Following the method described in Example 10, a formulation having the following composition is prepared:
naproxen coated granules (prepared in Example 6) 357 mg, sodium bicarbonate 52 mg, lactose 103.5 mg, saccarose 155 mg, ascorbic acid 52 mg, sodium sacchararinate 2.64 mg, flavour 12 mg, PVP K 30 65.86 mg and terbutyl alcohol 800 mg.

The sodium bicarbonate, lactose, saccarose, ascorbic acid and sodium saccharinate are sieved to obtain particles having dimensions of about 100 µm. The components are added to PVP solubilized in t-butyl alcohol. The coated microgranules of naproxen are suspended in the mixture. The mixture is then suspended in blister packets and lyophilized as described in Example 10.

## Claims

1. Prompt-release pharmaceutical composition for administration of an active ingredient which is released in an amount of no more than 10% by weight within 1 minute from mixing with the aqueous reconstitution phase and of at least 75% by its weight within 45 minutes from mixing with the aqueous reconstitution phase, said composition comprising:
a) a plurality of nuclei represented by microgranules having dimensions between 50 and 500 micrometers, containing at least one active ingredient and at least one pharmaceutically acceptable excipient;
b) a film coating applied onto said microgranules, consisting of a lipidic layer, sprayed in the melted state onto said microgranules, with no aid of solvents, said coating optionally comprising at least one hydrophilic additive, either incorporated into the lipidic layer or as separate hydrophilic layer applied onto said lipidic layer, provided that said coating contains 1%-25% by weight of lipid material with respect to the weight of microgranules to be coated;
c) a vehicle comprising one or more pharmaceutically acceptable excipients.

2. The pharmaceutical composition according to claim 1, for oral use.

3. The pharmaceutical composition according to claim 1, **characterized in that** the lipidic material is selected among: mono-, di- or tri- glycerides of fatty acids having from 6 to 32 carbon atoms, fatty acids having from 6 to 32 carbon atoms, fatty alcohols having from 6 to 32 carbon atoms, esters of propylene glycols or of sucrose with fatty acids having from 6 to 32 carbon atoms, waxes, and mixtures thereof.

4. The pharmaceutical composition according to claim 3, **characterized in that** the lipidic material is selected among monostearin, dipalmitin, tristearin, hydrogenated castor oil, stearic acid, cetyl alcohol, stearyl alcohol, propylene glycol monostearate, sucrose monostearate, sucrose monopalmitate, beeswax, white wax, candelilla wax, carnauba wax, and mixtures thereof.

5. The pharmaceutical composition according to claim 1, **characterized in that** the hydrophilic additive is selected among hydrophilic polymers and surfactant agents.

6. The pharmaceutical composition according to claim 5, **characterized in that** hydrophilic polymers are selected among cellulose acetophtalate, hydroxypropylmethylcellulose and polyethylene glycole, and surfactants are selected among dioctyl sodium solfosuccinate, sodium laurylsulfate, partial esters between C₆-C₃₂ fatty acids and anhydrides of sorbitol, and polyethylene ethers of C₆-C₃₂ fatty acids partial esters of sorbitol anhydrides.

7. The pharmaceutical composition according to claim 1, **characterized in that** the amount of hydrophilic additive is of 0.1%-5% by weight, with respect to the amount of lipidic material.

8. The pharmaceutical composition according to claim 1, **characterized in that** the lipidic material is a mixture comprising at least one mono-glyceride or di-glyceride of a fatty acid, at least one wax, and optionally at least one fatty alcohol.

9. The pharmaceutical composition according to claim 8, **characterized in that** the lipidic material comprises 40%-95% by weight of at least one mono- glyceride or di-glyceride of fatty acids, 5%-50% by weight of at least one wax, and optionally 0.5%-5% of at least a fatty alcohol, the percentages being referred to the total amount of lipidic mixture.

10. The pharmaceutical composition according to claim 8 or 9, **characterized in that** the mono-glyceride is glyceryl monostearate, the wax is selected among beeswax, white wax and camauba wax; the fatty alcohol is selected among cetyl alcohol, stearyl alcohol and mixtures thereof.

11. The pharmaceutical composition according to claim 8 or 9, **characterized in that** the lipidic material further comprises, as hydrophilic additive selected among hydrophilic polymer and surfactant agents, 1%-3% by weight of a hydrophilic polymer, or 2%-3% by weight of a surfactant.

12. The pharmaceutical composition according to claim 1, **characterized in that** the lipidic material comprises at least one triglyceride, and optionally about 0.5%-2% of a hydrophilic additive.

13. The pharmaceutical composition according to claim 12, **characterized in that** the tryglyceride is hydrogenated castor oil, and the additive is polyethylene glycole.

14. The pharmaceutical composition according to claim 1, **characterized in that** said nuclei are microgranules with a mean geometric diameter of 120-200 µm, with a standard deviation of 1.4-2.0, an aerated density of 0.4-0.7 g/ml, a packed density of 0.5-0.9 g/ml, an apparent density of 1.2-1.5 g/ml, a Carr Index of 5%-15%, and an angle of repose of 20°-40°.

15. The pharmaceutical composition according to claim 1, **characterized in that** nuclei are microgranules containing the active ingredient and a mixture of a filler and of a binder, and optionally also a plasticizing agent.

16. The pharmaceutically composition according to claim 15, **characterized in that** the filler is lactose, the binder is polyvinylpirrolidone, the plasticizing agent is polyethylene glycole.

17. The pharmaceutical composition according to claim 1, **characterized in that** the active ingredient is selected among those having unpleasant palatability or taste, poor stability in the administration vehicle, or hygroscopicity.

18. The pharmaceutical composition according to claim 1, **characterized in that** the active ingredient is selected among acetaminophen, naproxen, ibuprofen, diltiazem, moguisteine, potassium bicarbonate, diphenylhydramine hydrochloride or citrate, prednisone, fluoxetine, fluconazole, paroxetine, ketoprofen and dextromethorphane hydrobromide.

19. The pharmaceutical composition according to claim 1, **characterized in that** the vehicle comprises at least one excipient selected among suspending or structurating agents, sugars, buffering substances, flavors, edulcorants, substances to reinforce the taste, and any mixture thereof.

20. The pharmaceutical composition according to claim 1, **characterized in that** it further comprises an external phase comprising an aqueous phase, optionally added with one or more excipients.

21. Pharmaceutical composition according to claim 1, suitable for use in the preparation of an extemporaneous suspension.

22. Dosage unit form comprising a pharmaceutical composition according to claim 1, **characterized in that** it is a tablet or a solid aggregate.

23. Dosage unit form according to claim 22, **characterized in that** it is an effervescent tablet or solid aggregate.

24. Dosage form as claimed in claim 23, **characterized by** including an effervescent mixture which comprises bicarbonate, citric acid or tartaric acid.

25. Dosage form as claimed in anyone of claims 22 to 24, **characterized by** being lyophilized.

26. A process for preparing a prompt-release pharmaceutical composition as defined in claim 1, comprising atomizing a melted lipidic material onto the nuclei, using compressed preheated air as the atomization fluid, without the aid of any solvent.

27. The process as claimed in claim 26, **characterized in that** it is carried out in a fluidized-bed equipment.

28. The process as claimed in claim 26, **characterized in that** the lipidic material is melted at a temperature of 40°C-60°C above its melting point, sprayed onto the nuclei at a temperature of 20°C-30°C above its melting point, with a spraying rate of 2.0-4.0 grams/minute, using as atomization fluid compressed air preheated at a temperature of 40°C-60°C above said melting point, at a pressure of 2.5-3.5 bars.

29. The process according to claim 26, **characterized in that** it further comprises dispersing a hydrophilic additive into the melted lipidic material.

30. The process according to claim 26, **characterized in that** it further comprises applying a separate layer of hydrophilic material onto the lipidic layer.

31. A process for preparing a prompt-release pharmaceutical composition as defined in claim 1, comprising preparing said microgranules by wetting with a fluid a mixture of at least one active ingredient with at least one pharmaceutically acceptable excipeints in a high-shear mixer-granulator equipped with a mixer and a mill, and then kneading the mixture thus obtained, both the wetting and the kneading phase being carried out under the combined action of the mixer and of the mill.

32. The process according to claim 31, **characterized in that** the fluid is used in an amount of about 20-50 grams per kilogram of mixture of active ingredients and of excipients to be granulated.

33. The process according to claim 31, **characterized in that** granulation is carried out using 80-180 grams of fluid per Kg of mixture to be granulated, at a spray-rate of 10-40 grams/minute, at a spray-pressure of 1.5-2.5 bars, with a kneading time of 5-15 minutes, using a mixer speed of 50-600 rpm, and a mill speed of 1500-4000 rpm.

34. A prompt-release pharmaceutical composition comprising coated nuclei, obtained according to the process as defined in anyone of claims from 26 to 33.

35. Coated nuclei comprising nuclei and lipidic coating as defined in anyone of claims from 1 to 21.

36. Use of coated nuclei comprising nuclei and lipidic coating as defined in anyone of claims from 1 to 21, in the preparation of a prompt-release pharmaceutical composition useful for the extemporaneous preparation of liquid suspensions, of tablets or of solid aggregates.

## Patentansprüche

1. Pharmazeutische Zusammensetzung mit rascher Freisetzung zur Verabreichung eines Wirkstoffes, der in einer Menge von nicht mehr als 10 Gew.% innerhalb 1 Minute nach Mischen mit der wäßrigen Rekonstitutionsphase und von mindestens 75 % seines Gewichts innerhalb von 45 Minuten nach Mischen mit der wäßrigen Rekonstitutionsphase freigesetzt wird, wobei die Zusammensetzung umfaßt:
a) eine Vielzahl von durch Mikrokörnchen mit Abmessungen zwischen 50 und 500 µm dargestellten Kernen, die mindestens einen Wirkstoff und mindestens einen pharmazeutisch annehmbaren Exzipienten enthalten;
b) eine Überzugsschicht, die auf die Mikrokörnchen aufgetragen wird, bestehend aus einer lipidischen Schicht, die im geschmolzenen Zustand auf die Mikrokörnchen ohne Hilfe von Lösungsmitteln gesprüht wird, wobei die Beschichtung optional mindestens ein hydrophiles Additiv, entweder eingeschlossen in die lipidische Schicht oder als getrennte hydrophile Schicht aufgetragen auf die lipidische Schicht, umfaßt, vorausgesetzt, daß die Beschichtung 1 bis 25 Gew.% Lipidmaterial in Bezug auf das Gewicht der zu beschichtenden Mikrokörnchen enthält;
c) ein Vehikel umfassend ein oder mehrere pharmazeutisch annehmbare Exzipienten.

2. Pharmazeutische Zusammensetzung gemäß Anspruch 1 zur oralen Verwendung.

3. Pharmazeutische Zusammensetzung gemäß Anspruch 1, **dadurch gekennzeichnet, daß** das lipidische Material ausgewählt ist unter: Mono-, Di- oder Triglyceriden von Fettsäuren mit von 6 bis 32 Kohlenstoffatomen, Fettsäuren mit von 6 bis 32 Kohlenstoffatomen, Fettalkoholen mit von 6 bis 32 Kohlenstoffatomen, Estern von Propylenglycolen oder Saccharose mit Fettsäuren mit von 6 bis 32 Kohlenstoffatomen, Wachsen und Mischungen davon.

4. Pharmazeutische Zusammensetzung gemäß Anspruch 3, **dadurch gekennzeichnet, daß** das lipidische Material unter Monostearin, Dipalmitin, Tristearin, gehärtetem Rizinusöl, Stearinsäure, Cetylalkohol, Stearylalkohol, Propylenglycolmonostearat, Saccharosemonostearat, Saccharosemonopalmitat, Bienenwachs, weißem Wachs, Candelillawachs, Carnaubawachs und Mischungen davon ausgewählt ist.

5. Pharmazeutische Zusammensetzung gemäß Anspruch 1, **dadurch gekennzeichnet, daß** das hydrophile Additiv unter hydrophilen Polymeren und oberflächenaktiven Mitteln ausgewählt ist.

6. Pharmazeutische Zusammensetzung gemäß Anspruch 5, **dadurch gekennzeichnet, daß** die hydrophilen Polymere unter Celluloseacetophthalat, Hydroxypropylmethylcellulose und Polyethylenglycol ausgewählt sind, und die oberflächenaktiven Mittel unter Dioctylnatriumsulfosuccinat, Natriumlaurylsulfat, Partialestern zwischen C₆-C₃₂ Fettsäuren und Anhydriden von Sorbit und Polyethylenestern von C₆-C₃₂ Fettsäurepartialestern von Sorbitanhydriden ausgewählt sind.

7. Pharmazeutische Zusammensetzung gemäß Anspruch 1, **dadurch gekennzeichnet, daß** die Menge des hydrophilen Additivs von 0,1 bis 5 Gew.% in Bezug auf die Menge des lipidischen Materials ist.

8. Pharmazeutische Zusammensetzung gemäß Anspruch 1, **dadurch gekennzeichnet, daß** das lipidische Material eine Mischung umfassend mindestens ein Monoglycerid oder Diglycerid einer Fettsäure, mindestens ein Wachs und optional mindestens einen Fettalkohol ist.

9. Pharmazeutische Zusammensetzung gemäß Anspruch 8, **dadurch gekennzeichnet, daß** das lipidische Material 40 bis 95 Gew.% von mindestens einem Monoglycerid oder Diglycerid von Fettsäuren, 5 bis 50 Gew.% von mindestens einem Wachs und optional 0,5 bis 5 % von mindestens einem Fettalkohol umfaßt, wobei sich die Prozentzahlen auf die Gesamtmenge der lipidischen Mischung beziehen.

10. Pharmazeutische Zusammensetzung gemäß Anspruch 8 oder 9, **dadurch gekennzeichnet, daß** das Monoglycerid Glycerylmonostearat ist, das Wachs unter Bienenwachs, weißem Wachs und Carnaubawachs ausgewählt ist; der Fettalkohol unter Cetylalkohol, Stearylalkohol und Mischungen davon ausgewählt ist.

11. Pharmazeutische Zusammensetzung gemäß Anspruch 8 oder 9, **dadurch gekennzeichnet, daß** das lipidische Material weiter als hydrophiles Additiv ausgewählt unter hydrophilen Polymeren und oberflächenaktiven Mitteln 1 bis 3 Gew.% eines hydrophilen Polymers oder 2 bis 3 Gew.% eines oberflächenaktiven Mittels umfaßt.

12. Pharmazeutische Zusammensetzung gemäß Anspruch 1, **dadurch gekennzeichnet, daß** das lipidische Material mindestens ein Triglycerid und optional ungefähr 0,5 bis 2 % eines hydrophilen Additivs umfaßt.

13. Pharmazeutische Zusammensetzung gemäß Anspruch 12, **dadurch gekennzeichnet, daß** das Triglycerid gehärtetes Rizinusöl und das Additiv Polyethylenglycol ist.

14. Pharmazeutische Zusammensetzung gemäß Anspruch 1, **dadurch gekennzeichnet, daß** die Kerne Mikrokörnchen mit einem durchschnittlichen geometrischen Durchmesser von 120 bis 200 µm mit einer Standardabweichung von 1,4 bis 2,0, einer lufthaltigen Dichte von 0,4 bis 0,7 g/ml, einer gepackten Dichte von 0,5 bis 0,9 g/ml, einer Schüttdichte von 1,2 bis 1,5 g/ml, einem Carr-Index von 5 bis 15 % und einem Schüttwinkel von 20 bis 40° sind.

15. Pharmazeutische Zusammensetzung gemäß Anspruch 1, **dadurch gekennzeichnet, daß** die Kerne Mikrokörnchen enthaltend den Wirkstoff und eine Mischung eines Füllstoffes und eines Bindemittels und optional auch eines Weichmachers sind.

16. Pharmazeutische Zusammensetzung gemäß Anspruch 15, **dadurch gekennzeichnet, daß** der Füllstoff Lactose, das Bindemittel Polyvinylpyrrolidon, der Weichmacher Polyethylenglycol ist.

17. Pharmazeutische Zusammensetzung gemäß Anspruch 1, **dadurch gekennzeichnet, daß** der Wirkstoff unter denjenigen mit unangenehmer Schmackhaftigkeit oder unangenehmem Geschmack, schlechter Stabilität im Verabreichungsvehikel oder Hygroskopizität ausgewählt ist.

18. Pharmazeutische Zusammensetzung gemäß Anspruch 1, **dadurch gekennzeichnet, daß** der Wirkstoff unter Acetaminophen, Naproxen, Ibuprofen, Diltiazem, Moguistein, Kaliumbicarbonat, Diphenylhydraminhydrochlorid oder -citrat, Prednison, Fluoxetin, Fluconazol, Paroxetin, Ketoprofen und Dextromethorphanhydrobromid ausgewählt ist.

19. Pharmazeutische Zusammensetzung gemäß Anspruch 1, **dadurch gekennzeichnet, daß** das Vehikel mindestens einen Exzipienten ausgewählt unter suspendierenden oder strukturierenden Mitteln, Zuckern, Puffersubstanzen, Geschmacksstoffen, Süßmitteln, Substanzen um den Geschmack zu verstärken, und jeder Mischung davon umfaßt.

20. Pharmazeutische Zusammensetzung gemäß Anspruch 1, **dadurch gekennzeichnet, daß** sie weiter eine externe Phase umfassend eine wäßrige Phase, optional versetzt mit einem oder mehreren Exzipienten, umfaßt.

21. Pharmazeutische Zusammensetzung gemäß Anspruch 1, geeignet zur Verwendung bei der Herstellung einer unvorbereiteten Suspension.

22. Einheitsdosisform umfassend eine pharmazeutische Zusammensetzung gemäß Anspruch 1, **dadurch gekennzeichnet, daß** sie eine Tablette oder ein festes Aggregat ist.

23. Einheitsdosisform gemäß Anspruch 22, **dadurch gekennzeichnet, daß** sie eine sprudelnde Tablette oder ein sprudelndes festes Aggregat ist.

24. Dosisform gemäß Anspruch 23, **dadurch gekennzeichnet, daß** sie eine aufschäumende Mischung, die Bicarbonat, Zitronensäure oder Weinsäure umfaßt, einschließt.

25. Dosisform gemäß einem der Ansprüche 22 bis 24, **dadurch gekennzeichnet, daß** sie lyophilisiert ist.

26. Ein Verfahren zum Herstellen einer pharmazeutischen Zusammensetzung mit rascher Freisetzung gemäß Anspruch 1, umfassend Zerstäuben eines geschmolzenen lipidischen Materials auf den Kern, unter Verwenden von komprimierter vorgewärmter Luft als dem Zerstäubungsfluid ohne Hilfe eines Lösungsmittels.

27. Verfahren gemäß Anspruch 26, **dadurch gekennzeichnet, daß** es in einem Fließbettgerät durchgeführt wird.

28. Verfahren gemäß Anspruch 26, **dadurch gekennzeichnet, daß** das lipidische Material bei einer Temperatur von 40 bis 60°C überhalb seines Schmelzpunkts geschmolzen wird, bei einer Temperatur von 20 bis 30°C überhalb seines Schmelzpunkts mit einer Sprühgeschwindigkeit von 2,0 bis 4,0 g/Min. unter Verwenden von mit einer Temperatur von 40 bis 60°C überhalb des Schmelzpunkts vorgewärmter komprimierter Luft als Zerstäubungsfluid bei einem Druck von 2,5 bis 3,5 Bar auf die Kerne gesprüht wird.

29. Verfahren gemäß Anspruch 26, **dadurch gekennzeichnet, daß** es weiter Dispergieren eines hydrophilen Additivs in das geschmolzene lipidische Material umfaßt.

30. Verfahren gemäß Anspruch 26, **dadurch gekennzeichnet, daß** es weiter Auftragen einer getrennten Schicht aus hydrophilem Material auf die lipidische Schicht umfaßt.

31. Verfahren zum Herstellen einer pharmazeutischen Zusammensetzung mit rascher Freisetzung gemäß Anspruch 1, umfassend Herstellen der Mikrokörnchen durch Benetzen mit einem Fluid einer Mischung von mindestens einem Wirkstoff mit mindestens einem pharmazeutisch annehmbaren Exzipienten in einem Mischergranulator mit hohen Scherkräften, ausgestattet mit einem Mischer und einer Mühle, und dann Kneten der so erhaltenen Mischung, wobei sowohl die Benetzungs- als auch die Knetphase unter der kombinierten Wirkung des Mischers und der Mühle durchgeführt werden.

32. Verfahren gemäß Anspruch 31, **dadurch gekennzeichnet, daß** das Fluid in einer Menge von ungefähr 20 bis 50 g/kg der zu granulierenden Mischung der Wirkstoffe und Exzipienten verwendet wird.

33. Verfahren gemäß Anspruch 31, **dadurch gekennzeichnet, daß** die Granulierung unter Verwenden von 80 bis 180 g Fluid pro kg der zu granulierenden Mischung, mit einer Sprühgeschwindigkeit von 10 bis 40 g/Min., bei einem Sprühdruck von 1,5 bis 2,5 Bar, mit einer Knetzeit von 5 bis 15 Min. unter Verwenden einer Mischergeschwindigkeit von 50 bis 600 U/Min. und einer Mühlengeschwindigkeit von 1.500 bis 4.000 U/Min. durchgeführt wird.

34. Pharmazeutische Zusammensetzung mit rascher Freisetzung, umfassend beschichtete Kerne erhalten durch das Verfahren gemäß einem der Ansprüche 26 bis 33.

35. Beschichtete Kerne, umfassend Kerne und lipidische Beschichtung gemäß einem der Ansprüche 1 bis 21.

36. Verwendung von beschichteten Kernen, umfassend Kerne und lipidische Beschichtung gemäß einem der Ansprüche 1 bis 21, bei der Herstellung einer pharmazeutischen Zusammensetzung mit rascher Freisetzung, die für die unvorbereitete Herstellung von flüssigen Suspensionen, Tabletten oder festen Aggregaten nützlich ist.

## Revendications

1. Composition pharmaceutique à libération rapide pour l'administration d'un ingrédient actif qui est libéré en une quantité inférieure ou égale à 10% en poids en 1 minute par mélange avec la phase aqueuse de reconstitution et en une quantité supérieure à 75% en poids en 45 minutes par mélange avec la phase aqueuse de reconstitution, ladite composition comprenant :
a) une pluralité de noyaux représentés par des microgranulés de dimension comprise entre 50 et 500 micromètres, contenant au moins un ingrédient actif et au moins un excipient pharmaceutiquement acceptable ;
b) une pellicule enduite appliquée sur lesdits microgranulés, constituée d'une couche lipidique pulvérisée à l'état fondu sur lesdits microgranulés, sans l'aide d'aucun solvant, ladite pellicule comprenant optionnellement au moins un additif hydrophile, soit incorporé dans la couche lipidique, soit appliqué sous forme de couche hydrophile séparée sur ladite couche lipidique, sous réserve que ladite pellicule contienne de 1% à 25% en poids d'une matière lipidique par rapport au poids des microgranulés à enduire ;
c) un véhicule comprenant un ou plusieurs excipients pharmaceutiquement acceptables.

2. La composition pharmaceutique selon la revendication 1, pour usage oral.

3. La composition pharmaceutique selon la revendication 1, **caractérisée en ce que** la matière lipidique est choisie parmi : des mono-, di- ou tri-glycérides d'acides gras ayant de 6 à 32 atomes de carbone, des acides gras ayant de 6 à 32 atomes de carbone, des alcools gras ayant de 6 à 32 atomes de carbone, des esters de propylène glycols ou de sucrose et d'acides gras ayant de 6 à 32 atomes de carbone, des cires, et leurs mélanges.

4. La composition pharmaceutique selon la revendication 3, **caractérisée en ce que** la matière lipidique est choisie parmi la monostéarine, la dipalmitine, la tristéarine, l'huile de castor hydrogénée, l'acide stéarique, l'alcool cétylique, l'alcool stéarylique, le monostéarate de propylène glycol, le monostéarate de sucrose, le monopalmitate de sucrose, la cire d'abeille, la cire blanche, la cire de candelilla, la cire de carnauba, et leurs mélanges.

5. La composition pharmaceutique selon la revendication 1, **caractérisée en ce que** l'additif hydrophile est choisi parmi les polymères hydrophiles et les agents tensioactifs.

6. La composition pharmaceutique selon la revendication 5, **caractérisée en ce que** les polymères hydrophiles sont choisis parmi l'acétophtalate de cellulose, l'hydroxypropyl-méthylcellulose et le polyéthylène glycol, et les tensioactifs sont choisis parmi le dioctyl sulfosuccinate de sodium, le laurylsulfate de sodium, les esters partiels d'acides gras en C₆-C₃₂ et d'anhydrides de sorbitol, et les éthers de polyéthylène d'esters partiels d'acides gras en C₆-C₃₂ et d'anhydrides de sorbitol.

7. La composition pharmaceutique selon la revendication 1, **caractérisée en ce que** la quantité d'additif hydrophile est de 0,1%-5% en poids par rapport à la quantité de matière lipidique.

8. La composition pharmaceutique selon la revendication 1, **caractérisée en ce que** la matière lipidique est un mélange comprenant au moins un mono-glycéride ou di-glycéride d'acides gras, au moins une cire, et optionnellement au moins un alcool gras.

9. La composition pharmaceutique selon la revendication 8, **caractérisée en ce que** la matière lipidique comprend 40%-95% en poids d'au moins un mono-glycéride ou di-glycéride d'acides gras, 5%-50% en poids d'au moins une cire, et optionnellement 0,5%-5% en poids d'au moins un alcool gras, les pourcentages étant exprimés par rapport à la quantité totale de mélange lipidique.

10. La composition pharmaceutique selon la revendication 8 ou 9, **caractérisée en ce que** le mono-glycéride est le monostéarate de glycéryle, la cire est choisie parmi la cire d'abeille, la cire blanche et la cire de carnauba, l'alcool gras est choisi parmi l'alcool cétylique, l'alcool stéarylique et leurs mélanges.

11. La composition pharmaceutique selon la revendication 8 ou 9, **caractérisée en ce que** la matière lipidique comprend en outre comme additif hydrophile choisi parmi les polymères hydrophiles et les agents tensioactifs, 1%-3% en poids d'un polymère hydrophile ou 2%-3% en poids d'un tensioactif.

12. La composition pharmaceutique selon la revendication 1, **caractérisée en ce que** la matière lipidique comprend au moins un triglycéride, et optionnellement environ 0,5%-2% d'un additif hydrophile.

13. La composition pharmaceutique selon la revendication 12, **caractérisée en ce que** le triglycéride est de l'huile de castor hydrogénée et l'additif est le polyéthylène glycol.

14. La composition pharmaceutique selon la revendication 1, **caractérisée en ce que** lesdits noyaux sont des microgranulés ayant un diamètre géométrique moyen de 120-200 µm, avec un écart-type de 1,4-2,0, une densité aérée de 0,4-0,7 g/ml, une densité compactée de 0,5-0,9 g/ml, une densité apparente de 1,2-1,5 g/ml, un index de Carr de 5%-15%, et un angle de talus naturel de 20°-40°.

15. La composition pharmaceutique selon la revendication 1, **caractérisée en ce que** les noyaux sont des microgranulés contenant l'ingrédient actif et un mélange de charge et de liant, et optionnellement aussi un agent plastifiant.

16. La composition pharmaceutique selon la revendication 15, **caractérisée en ce que** la charge est le lactose, le liant est la polyvinylpyrrolidone, l'agent plastifiant est le polyéthylène glycol.

17. La composition pharmaceutique selon la revendication 1, **caractérisée en ce que** l'ingrédient actif est choisi parmi ceux ayant une sapidité ou un goût désagréable, une faible stabilité dans le véhicule d'administration ou une hygroscopicité.

18. La composition pharmaceutique selon la revendication 1, **caractérisée en ce que** l'ingrédient actif est choisi parmi l'acetaminophène, le naproxène, l'ibuprofène, le diltiazem, la moguistéine, le bicarbonate de potassium, le chlorhydrate ou le citrate de diphénylhydramine, la prednisone, le fluoxétine, le fluconazole, la paroxétine, le kétoprofène et le bromhydrate de dextrométhorphane.

19. La composition pharmaceutique selon la revendication 1, **caractérisée en ce que** le véhicule comprend au moins un excipient choisi parmi les agents de suspension ou structurants, les sucres, les substances tampons, les parfums, les édulcorants, les renforçateurs de goûts, et tout mélange de ces substances.

20. La composition pharmaceutique selon la revendication 1, **caractérisée en ce qu'**elle comprend en outre une phase externe comprenant un phase aqueuse, optionnellement additionnée d'un ou plusieurs excipients.

21. Composition pharmaceutique selon la revendication 1, adaptée à l'utilisation pour la préparation d'une suspension extemporanée.

22. Forme de dosage unitaire comprenant une composition pharmaceutique selon la revendication 1, **caractérisée en ce qu'**elle est un comprimé ou un agrégat solide.

23. Forme de dosage unitaire selon la revendication 22, **caractérisée en ce qu'**elle est un comprimé ou un agrégat solide effervescent.

24. Forme de dosage selon la revendication 23, **caractérisée par** l'incorporation d'un mélange effervescent qui comprend du bicarbonate, de l'acide citrique ou de l'acide tartarique.

25. Forme de dosage selon l'une quelconque des revendications 22 à 24, **caractérisée en ce qu'**elle est lyophilisée.

26. Procédé de préparation d'une composition pharmaceutique à libération rapide comme définie dans la revendication 1, comprenant l'atomisation d'une matière lipidique fondue sur les noyaux, en utilisant de l'air comprimé préchauffé comme fluide d'atomisation, sans l'aide d'aucun solvant.

27. Le procédé selon la revendication 26, **caractérisé en ce qu'**il est effectué dans un équipement à lit fluidisé.

28. Le procédé selon la revendication 26, **caractérisé en ce que** la matière lipidique est fondue à une température de 40°C-60°C au-dessus de son point de fusion, pulvérisée sur les noyaux à une température de 20°C-30°C au-dessus de son point de fusion, avec un débit de pulvérisation de 2,0-4,0 g/minute en utilisant comme fluide d'atomisation de l'air comprimé préchauffé à une température de 40°C-60°C au-dessus dudit point de fusion, à une pression de 2,5-3,5 bars.

29. Le procédé selon la revendication 26, **caractérisé en ce qu'**il comprend en outre la dispersion d'un additif hydrophile dans la matière lipidique fondue.

30. Le procédé selon la revendication 26, **caractérisé en ce qu'**il comprend en outre l'application d'une couche séparée de matière hydrophile sur la couche lipidique.

31. Procédé de préparation d'une composition pharmaceutique à libération rapide comme définie dans la revendication 1, comprenant la préparation desdits microgranulés par mouillage avec un mélange fluide d'au moins un ingrédient actif avec au moins un excipient pharmaceutiquement acceptable dans un mélangeur-granulateur à fort cisaillement équipé d'un mélangeur et d'un moulin, et ensuite le malaxage du mélange ainsi obtenu, les deux phases de mouillage et de malaxage étant effectuées sous l'action combinée du mélangeur et du moulin.

32. Le procédé selon la revendication 31, **caractérisé en ce que** le fluide est utilisé en une quantité d'environ 20-50 grammes par kilogramme de mélange d'ingrédients actifs et d'excipients à granuler.

33. Le procédé selon la revendication 31, **caractérisé en ce que** la granulation est effectuée en utilisant 80-180 grammes de fluide par kilo de mélange à granuler, avec un débit de pulvérisation de 10-40 grammes/minute, à une pression de pulvérisation de 1,5-2,5 bars, avec un temps de malaxage de 5-15 minutes, en utilisation une vitesse de mélangeur de 50-600 trs/min et une vitesse de moulin de 1500-4000 trs/min.

34. Composition pharmaceutique à libération rapide comprenant des noyaux enrobés obtenus par le procédé défini dans l'une quelconque des revendications 26 à 33.

35. Noyaux enrobés comprenant des noyaux et des revêtements lipidiques comme définis dans l'une quelconque des revendications 1 à 21.

36. L'utilisation de noyaux enrobés comprenant des noyaux et un revêtement lipidique comme défini dans l'une quelconque des revendications 1 à 21, dans la préparation d'une composition pharmaceutique à libération rapide utile pour la préparation extemporanée de suspensions liquides, de comprimés ou d'agrégats solides.
